(19)

Europäisches
Patentamt
European
Patent Office
Office européen
des brevets

(11)  **EP 4 082 438 B1**

(12)  **EUROPEAN PATENT SPECIFICATION**

(45) Date of publication and mention
of the grant of the patent:
**16.07.2025  Bulletin 2025/29**

(21) Application number: **20907329.5**

(22) Date of filing: **22.12.2020**

(51) International Patent Classification (IPC):
*A61B 5/397* (2021.01)    *A61B 5/22* (2006.01)
*G16H 50/20* (2018.01)    *G16H 50/30* (2018.01)
*G16H 20/40* (2018.01)

(52) Cooperative Patent Classification (CPC):
**A61B 5/228; A61B 5/397; A61B 5/4542;
A61B 5/7242; A61B 5/7246; A61B 5/725;
A61B 5/7257; A61B 5/7267; G16H 50/20;
G16H 50/30;** G16H 20/40

(86) International application number:
**PCT/JP2020/048015**

(87) International publication number:
**WO 2021/132269 (01.07.2021 Gazette 2021/26)**

(54) **CHEWING ASSISTANCE SYSTEM**

KAUHILFESYSTEM

SYSTÈME D'ASSISTANCE À LA MASTICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **26.12.2019   JP 2019237303**

(43) Date of publication of application:
**02.11.2022   Bulletin 2022/44**

(73) Proprietor: **SUNSTAR INC.
Takatsuki-shi, Osaka 569-1195 (JP)**

(72) Inventor: **KANATA, Takeshi
Takatsuki-shi, Osaka 569-1195 (JP)**

(74) Representative: **Vossius & Partner
Patentanwälte Rechtsanwälte mbB
Siebertstraße 3
81675 München (DE)**

(56) References cited:
**WO-A1-2018/146672      WO-A1-2019/230528
JP-A- 2019 052 901      JP-A- 2019 205 780
US-A1- 2017 265 978      US-A1- 2021 212 621**

• **KUROSAWA, HIROKI ET AL.: "Cami-log :
Proposal of Application to Improve Daily
Chewing Activities using Myoelectric
Information", PROCEEDINGS OF IPSJ
INTERACTION 2017, 23 February 2017
(2017-02-23), pages 907 - 912, XP055943841**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

## Description

### TECHNICAL FIELD

The present invention relates to a system for assisting in health maintenance and promotion in an oral cavity and a throat region in order to extend healthy life expectancy, and particularly relates to a system for assisting in and supporting improvement of quality of chewing as an "function of chewing and eating deliciously".

### BACKGROUND ART

Chewing food, swallowing behavior, salivation, and the like exert a great influence on the brain and the whole body, and exert a great influence on physical and mental health and healthy life expectancy. Health maintenance and enhancement in an oral cavity and a throat region are considered to consequently extend healthy life expectancy.

Particularly, sufficient chewing of solid meals is considered to lead to promotion of physical and mental growth, brain activation, enhancement of motor function, obesity inhibition, aging prevention, and sociality maintenance, to exhibit an effect of extending healthy life expectancy. Insufficient chewing such as the small number of chewing times in intake of a meal leads to deterioration of a chewing function of a growing child, and oral frailty of elderly people (see Non-patent Literature 1).

Furthermore, "partial chewing" in which chewing is performed always on the same side affects teeth and the jaw, the face, and the like to, for example, shorten lifetimes of teeth on one side, easily soil teeth by which chewing is not performed, apply a load onto the jaw joint, or distort the face, and also affects the whole body to result in, for example, distortion of the body or stiff shoulders or low back pain. Balance (occlusal interference) in occlusion between the left side and the right side is also considered to be associated with physical and affective stress, and affects both sympathetic nerve and parasympathetic nerve functions.

A device such as an electromyograph for counting the number of chewing times and a device for numerically indicating an occlusal force have been provided for measuring chewing quality. However, a simple system that can accurately obtain in detail quality of complicated chewing behavior having complex aspects, has not been provided.

PTL 3 relates to a method for calculating eating bites of a user. PTL 4 relates to a learning model generation apparatus for determining the masticatory side of a user. PTL 5 relates to a device for measuring dental occlusion of a subject by capacitive measurement.

### CITATION LIST

### [PATENT LITERATURE]

[PTL 1] Japanese Unexamined Patent Application Publication No. H6-98865
[PTL 2] Japanese Unexamined Patent Application Publication No. 2019-47859
[PTL 3] WO 2018/146672 A1
[PTL 4] WO 2019/230528 A1
[PTL 5] US 2017/265978 A1

### [NON PATENT LITERATURE]

[NPL 1] Kobayashi Yoshinori, irai ronbun, Kogo/soshaku ga tsukuru kenkojumyo, Nichihotetsukaishi Ann Jpn Prosthodont, Soc3, p189-219, 2011 (Yoshinori Kobayashi, solicited article, A long Life Built by Mastication and Occlusion, Annals of Japan Prosthodontic Society, Soc3, p189-219, 2011)

### SUMMARY OF THE INVENTION

### PROBLEMS TO BE SOLVED BY THE INVENTION

Therefore, the present invention has been made in order to overcome the aforementioned circumstances, and an object of the present invention is to provide a simple system that can accurately obtain in detail quality of complicated chewing behavior having complex aspects, and that is a chewing assistance system capable of accurately supporting improvement of chewing quality, and health maintenance and promotion.

### SOLUTION TO THE PROBLEMS

In view of the aforementioned circumstances, the inventor of the present invention has found, as a result of thorough study, that chewing behavior and chewing quality can be accurately analyzed and determined in detail by, for example, frequency-analyzing a muscle activity signal obtained by an electromyograph or the like during a meal and utilizing a power value in a specific frequency band that is dominant particularly in activity during chewing, and assistance in improvement of chewing quality and health maintenance and promotion can be performed based on the determination result, to complete the present invention.

That is, the present invention is specified by the independent claims. Preferred embodiments are defined in the dependent claims. In the following description, although numerous features may be designated as optional, it is nevertheless acknowledged that all features comprised in the independent claims are not to be read as optional.

ADVANTAGEOUS EFFECTS OF THE INVENTION

According to the present invention described above, a muscle activity signal is frequency-analyzed, chewing behavior is analyzed based on the frequency-analyzed muscle activity signal, quality of the chewing behavior is determined, and assistance information corresponding to the determined chewing quality can be provided. Therefore, a simple system that can obtain in detail quality of complicated chewing behavior having complex aspects can be provided to accurately support improvement of chewing quality, and health maintenance and promotion.

The present invention having such a configuration can provide the system that can accurately provide growth information about quality of healthy chewing for growing children, and that contributes to healthy development of a chewing action function for the growing children. Also for elderly people, the present invention can provide the system that can accurately provide assistance information corresponding to chewing quality and that contributes to maintenance and enhancement of a chewing action function for elderly people

BRIEF DESCRIPTION OF THE DRAWINGS

[FIG. 1] FIG. 1 is a block diagram illustrating a configuration of a chewing assistance system according to a representative embodiment of the present invention.
[FIG. 2A] FIG. 2A illustrates raw data of a muscle activity signal of masticatory muscle on the left side.
[FIG. 2B] FIG. 2B illustrates raw data of a muscle activity signal of masticatory muscle on the right side.
[FIG. 3A] FIG. 3A illustrates a heat map representing a frequency distribution for a muscle activity power value on the left side.
[FIG. 3B] FIG. 3B illustrates a heat map representing a frequency distribution for a muscle activity power value on the right side.
[FIG. 4] FIG. 4 is an explanatory diagram illustrating a method for determining a chewing section from an envelope.
[FIG. 5] FIG. 5 shows a graph representing raw data of a muscle activity signal of masticatory muscle and an envelope obtained by FFT processing performed on the raw data.
[FIG. 6] FIG. 6 shows a graph representing a value of surface integral of each of raw data and an envelope in a chewing section, and an occlusal force in the chewing section.
[FIG. 7] FIG. 7 shows graphs representing raw data of muscle activity signals on the left side and the right side in the case of chewing being performed predominantly by left-side teeth in an unbalanced manner and envelopes obtained by FFT processing performed on the raw data.
[FIG. 8] FIG. 8 shows graphs representing raw data of muscle activity signals on the left side and the right side in the case of chewing being performed predominantly by right-side teeth in an unbalanced manner and envelopes obtained by FFT processing performed on the raw data.
[FIG. 9] FIG. 9 shows graphs representing raw data of muscle activity signals of temporal muscle and masseter in the case of chewing being performed by back teeth, and envelopes obtained by FFT processing performed on the raw data.
[FIG. 10] FIG. 10 shows graphs representing raw data of muscle activity signals of temporal muscle and masseter in the case of chewing being performed by anterior teeth, and envelopes obtained by FFT processing performed on the raw data.
[FIG. 11A] FIG. 11A shows a graph representing raw data of a muscle activity signal at various chewing speeds and rhythms, and an envelope obtained by FFT processing performed on the raw data.
[FIG. 11B] FIG. 11B shows a graph representing raw data of a muscle activity signal at various chewing speeds and rhythms, and an envelope obtained by FFT processing performed on the raw data.
[FIG. 12] FIG. 12 is a flowchart showing a procedure of processing performed by the chewing assistance system according to the representative embodiment.

DESCRIPTION OF EMBODIMENTS

Next, an embodiment of the present invention will be described in detail with reference to the accompanied drawings.

Chewing during a meal is associated with a person's favorite hardness/softness of food, a motion of biting through and masticating the food, the number of times of chewing the food, a chewing time, rhythm, and the like. Balance between chewing teeth is also among them. For determining chewing quality based on whether or not such a function of chewing and eating deliciously is proper, the system of the present invention frequency-analyzes a muscle activity signal of masticatory muscle, and analyzes chewing behavior such as the number of chewing times, chewing rhythm, transition of occlusal actions, an occlusal force, chewing balance between a left side and a right side, unbalanced diet, and a way of using masseter, so as to determine the chewing quality, and to indicate change with the elapse of time according to difference between the past state and the present state, for example. Therefore, the system of the present invention can present an improving state of the chewing quality.

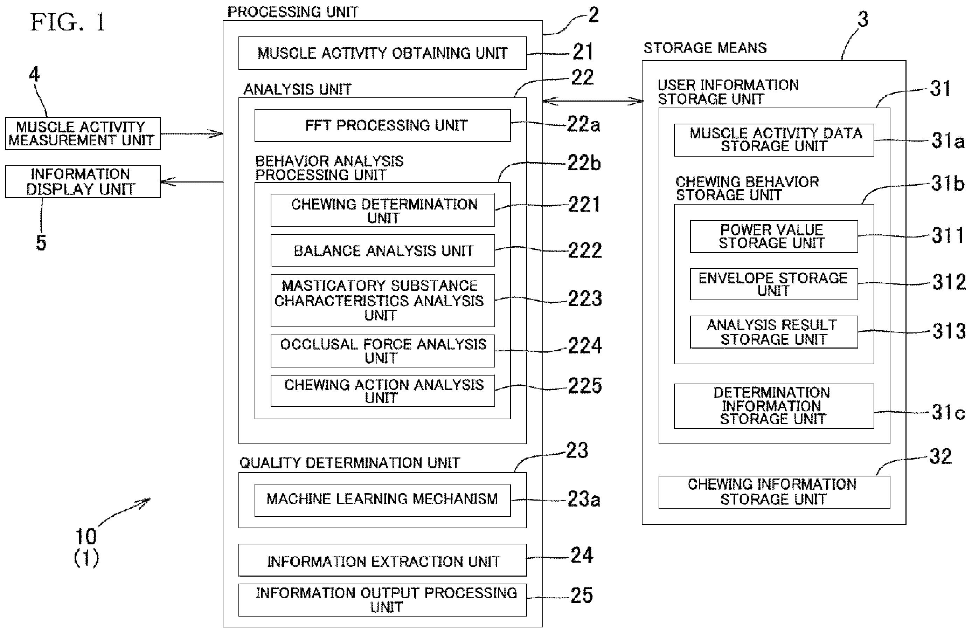

Specifically, as shown in FIG. 1, a chewing assistance system 1 of the present invention is configured by a single or a plurality of information processing devices 10 each including a processing unit 2, storage means 3, a muscle activity measurement unit 4, and an information display unit 5. Specifically, the information processing device 10 is implemented by, for example, a computer that includes the processing unit 2 as a main unit and also includes, for example, storage means, input means such as a pointing device, a keyboard, and a touch panel, display means such as a display, and another unit such as a communication controller which is not illustrated.

The processing unit 2 includes a CPU such as a microprocessor as a main unit and also has a not-illustrated storage unit, such as a RAM and a ROM, in which a program for providing procedures of various processing operations, and process data are stored. The storage means 3 includes a memory, a hard disk, and the like disposed inside and/or outside the information processing device 10. A part or all of contents in the storage unit may be stored in, for example, a hard disk or a memory of another computer that is connected to the information processing device 10 so as to be communicable with each other. The information processing device having such a configuration may be a dedicated device that is installed in a dental clinic, a hospital, another institution, a store, or the like, or may be a general-purpose household personal computer. The information processing device may be, for example, a smartphone carried by a user.

The processing unit 2 includes, as its functions, a muscle activity obtaining unit 21 as muscle activity obtaining means, an analysis unit 22, a quality determination unit 23 as quality determination means, an information extraction unit 24, and an information output processing unit 25. The muscle activity obtaining unit 21 obtains a muscle activity signal, of masticatory muscle of a user, which is obtained and transmitted by the muscle activity measurement unit 4, and stores the muscle activity signal in a muscle activity data storage unit 31a of a user information storage unit 31. The analysis unit 22 frequency-analyzes the muscle activity signal, analyzes chewing behavior based thereon, and stores information of the analyzed chewing behavior in a chewing behavior storage unit 31b of the user information storage unit 31. The quality determination unit 23 determines chewing quality based on the information of the chewing behavior, and stores information of the determined chewing quality in a determination information storage unit 31c of the user information storage unit 31. The information extraction unit 24 receives input of the information of the determined chewing quality, and extracts information to be recommended from information, about the chewing quality, stored in a chewing information storage unit 32. The information output processing unit 25 presents the information to the user by, for example, displaying the information on a display (information display unit 5). These processing functions are executed by the above-described program.

The muscle activity measurement unit 4 corresponds to an electromyograph or the like, and preferably includes communication means capable of performing short-range radio transmission and reception of data to and from a smartphone of a user as the information processing device 10. The muscle activity measurement unit 4 also corresponds to, for example, an external electromyograph that is wired-connected or wirelessly connected to, for example, a dedicated computer device as the information processing device 10. A muscle activity signal of masticatory muscle of a user is obtained by the muscle activity measurement unit 4.

The muscle activity measurement unit 4 measures a muscle activity of at least one of four masticatory muscles which are temporal muscles and masseters on both sides of a head portion in order to obtain the muscle activity signal. At least two muscle activities to be compared with each other are measured in order to measure balance during chewing. That is, muscle activities of at least left and right temporal muscles or at least left and right masseters are to be obtained in order to measure balance between the left side and the right side. Muscle activities of at least temporal muscle and masseter on the left side or at least temporal muscle and masseter on the right side are to be obtained in order to measure balance between the anterior side and the posterior side.

The analysis unit 22 functions as analysis means, and frequency-analyzes the muscle activity signal obtained by the muscle activity obtaining unit 21, and analyzes chewing behavior based on a change state of a power value in a specific frequency band. The analysis can be more accurately performed by thus utilizing the power value in the specific frequency band (for example, 150 Hz to 450 Hz) that is particularly dominant in activity during chewing.

More specifically, an FFT processing unit 22a and a behavior analysis processing unit 22b are provided. The FFT processing unit 22a obtains data of the muscle activity signal from the muscle activity data storage unit 31a, performs fast Fourier transform for each block, obtains an average power value in a specific frequency band, and stores the power value in a power value storage unit 311, and further generates an envelope of the obtained power value (hereinafter, simply referred to as "envelope" in the description herein), and stores the envelope in an envelope storage unit 312. The behavior analysis processing unit 22b analyzes the chewing behavior and stores the result in an analysis result storage unit 313.

A specific example of the process performed by the FFT processing unit 22a is as follows. The process is performed on the assumption that the muscle activity measurement unit is a device that performs sampling at 2000 samples/second. Firstly, the FFT processing unit 22a divides raw data (2000 samples/second) of the muscle activity signal into blocks each including a predetermined number of samples (64 samples in this example) and performs fast Fourier transform for each block.

In this example, in the fast Fourier transform for each block, 0 to 1000 Hz is equally divided into 32 to set 32 pins (frequency), and a power value for each predetermined number of pins is calculated. Each pin represents a frequency (integer multiple) for each 31.25 Hz. The FFT processing unit 22a further calculates an average value of, for example, eight power values in a specific frequency band (between 7 pin and 14 pin, that is, between 218.75 and 437.5 Hz in this example) for each block, and outputs the average value as an average power value of each block. The power value represents amplitude of a frequency spectrum at a specific frequency.

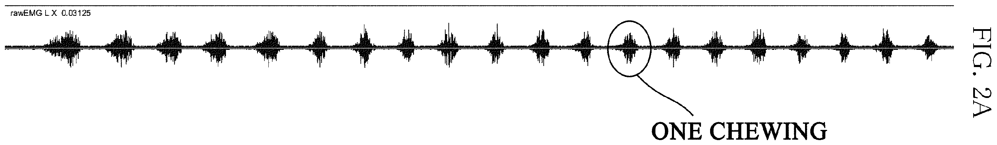

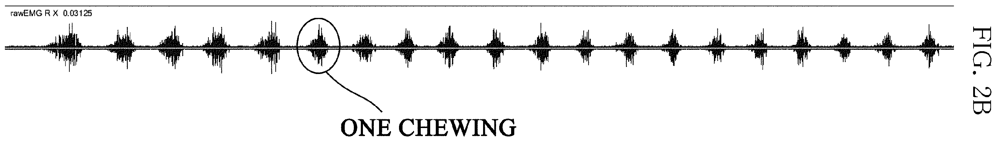

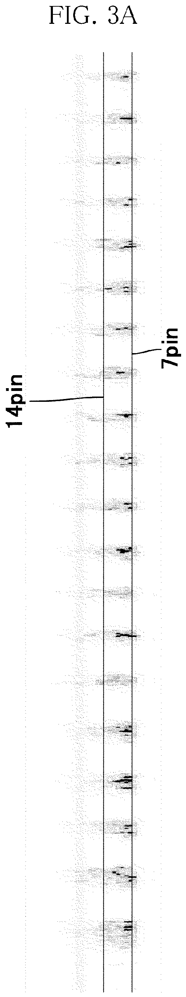

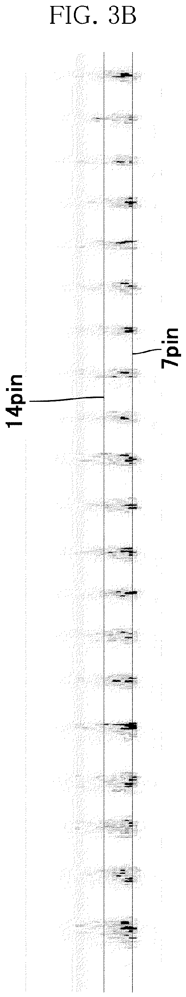

FIG. 2A and FIG. 2B show raw data (2000 samples/second) of muscle activity signals of left and right masticatory muscles. In FIG. 3A and FIG. 3B, a frequency distribution for a power value obtained through the fast Fourier transform of the raw data for each block by the FFT processing unit 22a according to the above-described specific example is confirmed in a heat map. FIG. 2A and FIG. 3A show the data/heat map for the left side. FIG. 2B and FIG. 3B show the data/heat map for the right side. According to the heat map, in a case where the average value of the power values is obtained between 7 pin and 14 pin, the chewing behavior can be more accurately determined from the muscle activity signal. However, the pin range may be other than "between 7 and 14". For example, "between 6 and 14" or "between 6 and 15" may also be a preferable pin range.

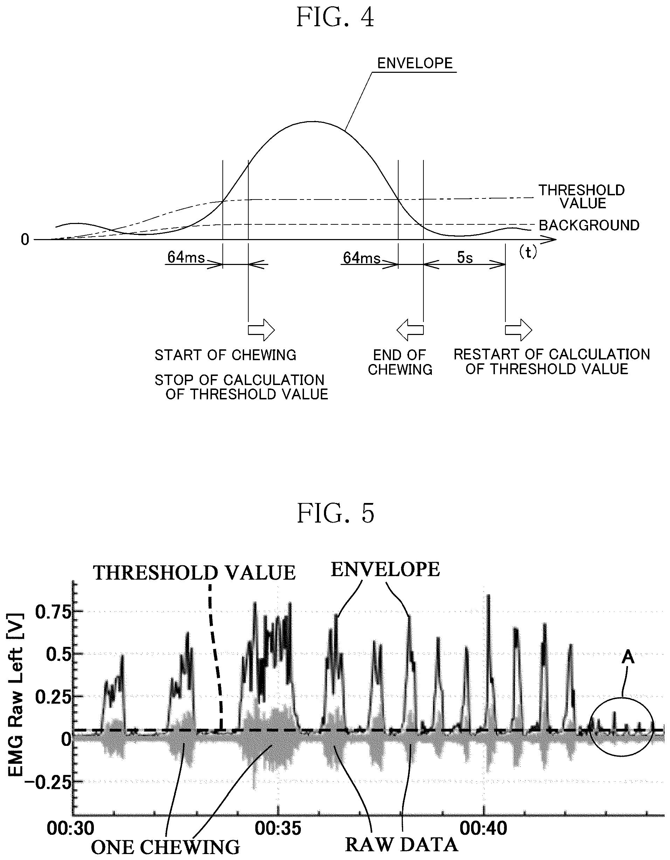

For example, FIG. 5 shows an example of an envelope that is obtained by connecting the average power values, of the respective blocks (for each 32 ms), outputted through the fast Fourier transform of the raw data by the FFT processing unit 22a according to the above-described example. By this envelope, the graph more accurately represents the chewing behavior as compared with a graph of the raw muscle activity data. When this envelope is used, the chewing behavior can be more accurately determined by using data in a frequency band for chewing.

The muscle activity data (raw data) stored in the muscle activity data storage unit 31a is preferably deleted from the storage means 3 at a time when the analysis result is stored in the analysis result storage unit 313 from the viewpoint of reducing a storage region.

The behavior analysis processing unit 22b analyzes various chewing behaviors by, for example, using such an envelope generated by the FFT processing unit 22a, and stores the chewing behaviors in the analysis result storage unit 313. The analyzed chewing behaviors represent, for example, the number of chewing times, chewing rhythm, transition of occlusal actions during a meal, an occlusal force level, chewing balance between the anterior and posterior sides/between the left and right sides, and characteristics of a masticatory substance. In this example, the analysis of the chewing behavior is performed on the assumption that the behavior analysis processing unit 22b includes a chewing determination unit 221 for determining whether or not the chewing is performed. The chewing determination unit 221 determines that chewing is performed when the envelope indicates a value that exceeds a predetermined threshold value. Specifically, the chewing is determined as follows.

(Chewing determination)

Preferably, a background is firstly calculated from an envelope, among envelopes, in a definite non-chewing section in which a muscle activity (average power value) is small and stable, and a value obtained by multiplying the background by a coefficient is set as a threshold value for determining the chewing. When the threshold value is exceeded under a certain condition, chewing is determined to be performed.

Specifically, the value of the background can be firstly calculated through low pass filter processing of the envelope. The filter may be a primary autoregressive filter represented by the following equation.

$$Y_n = 0.99Y_{n-1} + 0.01X_{n-80}$$

"$X_{n-80}$" represents a value of an envelope that has been obtained 2.56 seconds earlier. In this equation, "2.56" represents a value obtained as 80 samples/31.25 samples/s = 2.56s. "$Y_{n-1}$" represents the latest value of the background level, and "$Y_n$" represents a new value of the background level. "0.99" represents a filter constant, and "0.01" is for ensuring the total gain with a gain factor (gain coefficient) of an input signal.

The calculation is preferably performed by integers in order to reduce a calculation load on a built-in processor. This can be performed by performing multiplication of a value from FFT algorithm at a magnification of 10000 (8 bit algorithm). Furthermore, the above-described filter is obtained by the following equation.

$$Y_n = (99Y_{n-1} + X_{n-80})/100$$

Preferably, the value of the background is not calculated until a predetermined time elapses from detection of the end of chewing after detection of the start of the chewing, and the background level obtained before the detection of the start of the chewing is maintained.

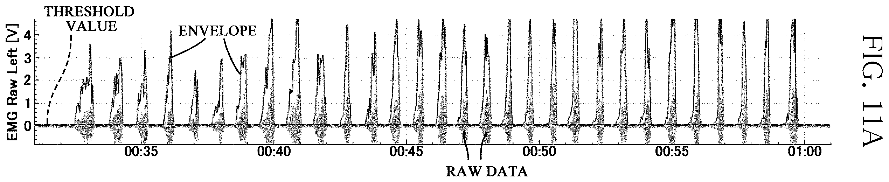

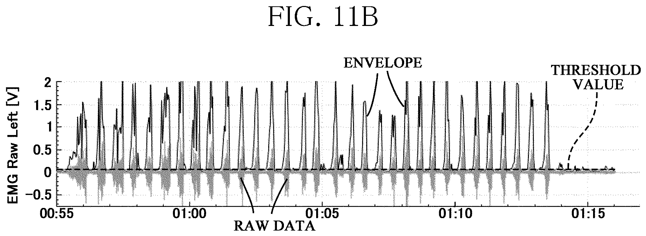

As shown in FIG. 4, the threshold value is set as a value (for example, a value that is 2.6 times the background level) obtained by multiplying the background level by a predetermined value. The start/the end of chewing are preferably detected when the envelope is greater than/less than the threshold value for two sample times (64 ms), respectively. The chewing determination unit 221 determines whether or not the chewing is performed in, for example, such a manner. According thereto, information about the number of chewing times, the chewing speed, and rhythm can be analyzed. As shown in FIG. 11A and FIG. 11B, the chewing speed and rhythm vary.

As another method for determining whether or not chewing is performed, a method in which the background is set as a value of a moving average of the envelopes in a certain section as represented by the following equation, is considered. Similarly to the above-described calculation of the background, a value that has been obtained 2.56 seconds earlier (80 samples) is set as a threshold value for the present time, and a background threshold value to be adopted for the average value can be set (for example, 1.2 times).

$$Y_n = X_{n-80} + 4\sigma_{n-80}$$

In the equation, "$Y_n$" represents a new value of the background level, and $X_{n-80}$ represents a value of a moving average of an envelope which has been obtained 2.56 seconds earlier. "2.56" represents a value obtained as 80 samples/31.25 samples/s = 2.56s. The value of the moving average represents an envelope obtained before a time point of the calculation (10 samples/31.25 samples/s = 320 ms earlier), "$\sigma$" represents a standard deviation, and "4" represents a deviation coefficient.

In this case, also for a threshold value for determining chewing, for example, a value obtained by multiplying the background average value and the deviation by a predetermined deviation coefficient (for example, 4) is preferably set as the threshold value.

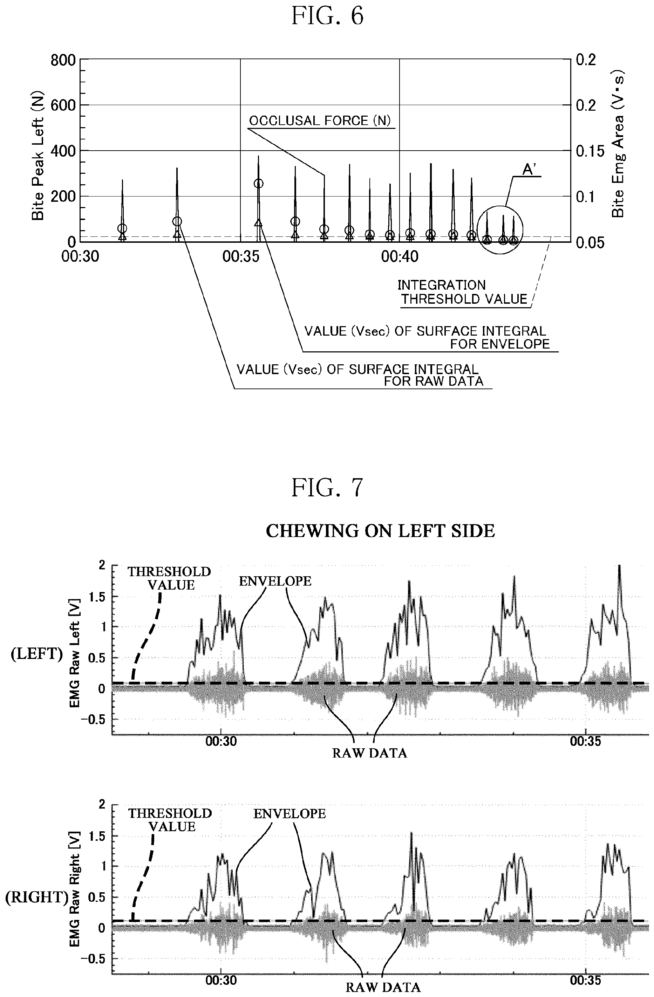

Although the start of chewing is detected when the envelope indicates a value greater than the threshold value for a predetermined time period, it is also preferable as an embodiment that, as shown in FIG. 6, a value of surface integral for an envelope in the chewing section is further calculated, and an integration threshold value is also set, and when an integral value of the envelope in one chewing section in which chewing is detected is less than the integration threshold value, the section is not determined as a chewing section and is excluded from the chewing. Thus, a short weak section (a section surrounded as A in FIG. 5, a section surrounded as A' in FIG. 6) among the chewing sections determined in FIG. 5 is excluded from the chewing, and only a definite chewing action can be determined as chewing, and counted.

In the "background", vibration is repeated in a certain range even in a state where a muscular action of a human body does not occur, and a potential (noise) in the resting state is "background (noise)". However, as in the present invention, in the chewing activity, a muscle activity which does not exceed the threshold value for determination occurs due to human reaction (shake the head, have an intake of breath) other than chewing, and when such a muscle activity occurs over a certain time period, the threshold value is increased by using only the above-described method for calculating the threshold value, and the determination of chewing may be hindered depending on the degree.

Specifically, since the calculation of the threshold value is stopped only when the muscle activity indicates a value that exceeds the threshold value, a muscle activity event determination threshold value is increased due to the following two influences. (1) a case where, although a muscle activity event indicates a value exceeding the threshold value for a moment, the duration is short and this is not counted as the event, and (2) a case where a small-scale muscle activity occurs for a short time period. The increase of the threshold value in these cases may lead to erroneous determination with respect to chewing to be actually counted and causes chewing strength to be calculated as an excessively small value.

Therefore, the threshold value is preferably calculated as follows. That is, a fluctuation range of an envelope in a certain time period is calculated, and if abrupt change occurs such that the fluctuation range exceeds a predetermined value, calculation of the threshold value is stopped in the section. Meanwhile, if the fluctuation is maintained so as to be gentle such that the fluctuation range of an envelope in a certain time period does not exceed the predetermined value, calculation of the threshold value is performed in the section. Thus, increase of the threshold value due to the influence in the above-described case (1) or (2) is inhibited, and a stable threshold value can be obtained, and the muscle activity event determination threshold value can accurately follow gentle increase of a myogenic potential and increase of a myogenic potential over a long time period.

(Balance analysis)

In the present embodiment, the behavior analysis processing unit 22b further includes a balance analysis unit 222 for analyzing chewing balance among the anterior, the posterior, the left, and the right sides. For example, for chewing balance between the left side and the right side, the balance analysis unit 222 calculates one or both of the maximum peak value and an integral value of each of envelopes that are generated in the same one chewing section from muscle activity data of the same masticatory muscles (temporal muscles/masseters) on the left side and the right side, and performs comparison to determine difference between the left-side chewing force and the right-side chewing force. In a case where the difference in force exceeds a predetermined threshold value, or in a case where such chewing continues a plural number of times, chewing is determined to be performed predominantly on the left side or the right side in an unbalanced manner.

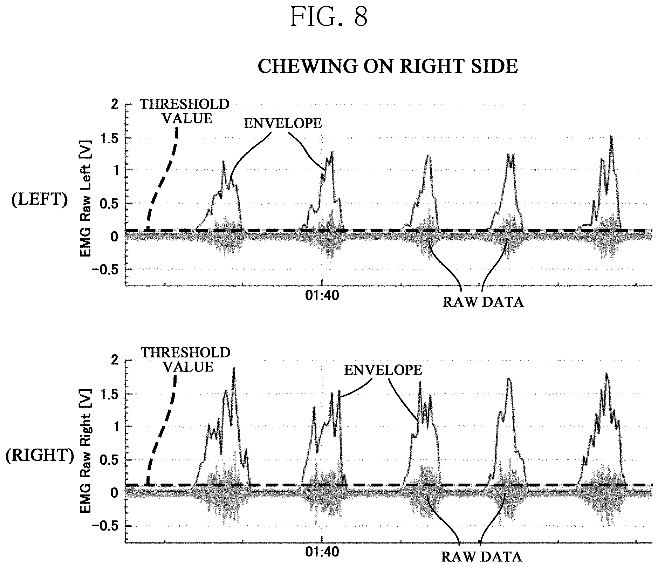

FIG. 7 shows envelopes on the left side and the right side in the case of chewing being performed predominantly on the left side in an unbalanced manner. As is apparent from FIG. 7, both an integral value and a peak value in each chewing section indicate greater values on the left side. Meanwhile, FIG. 8 shows envelopes in the case of chewing being performed predominantly on the right side in an unbalanced manner. Both an integral value and a peak value indicate greater values on the right side. This indicates that, by comparing the integral value or the maximum peak value of the envelope between the left side and the right side in a chewing section, balance between the left side and the right side can be analyzed.

Even in partial chewing in which food is continuously bit by teeth on one side, the muscle activities on the left side and the right side indicate almost the same tendencies in masticatory muscle (temporal muscle and masseter) in a healthy condition. Therefore, it is difficult to obtain difference in predominance between the left side and the right side directly from an original signal (raw data) of the muscle activity. In the balance analysis using frequency analysis as in the present embodiment, one, of muscles on the left side and the right side, which is predominantly used can be accurately determined in the analysis.

For chewing balance between the anterior side and the posterior side, the maximum peak values of the envelopes which are generated in the same one chewing section from muscle activity data of temporal muscle and masseter are calculated and compared with each other. If the peak value of the temporal muscle is less than the peak value on the masseter side by a predetermined threshold value or more, or if such chewing continues a plural number of times, chewing can be determined to be performed predominantly on the anterior teeth side in an unbalanced manner.

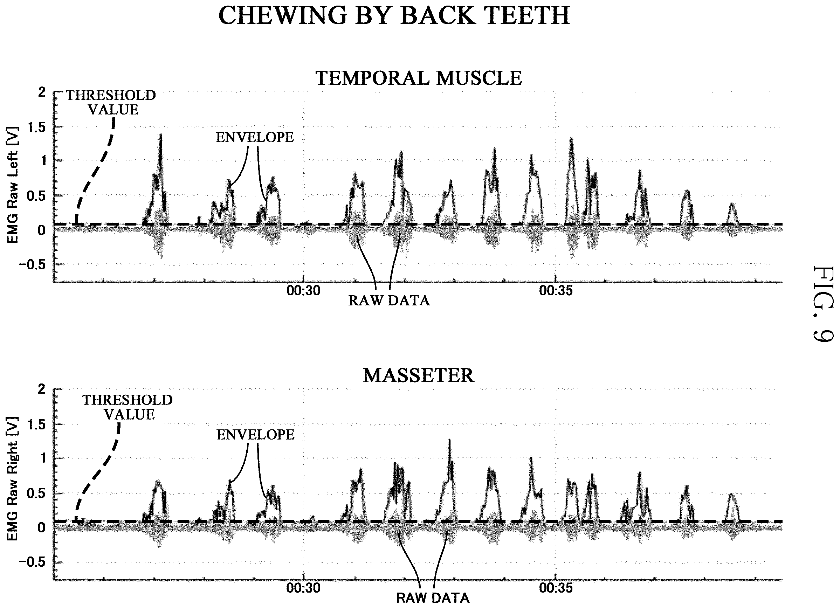

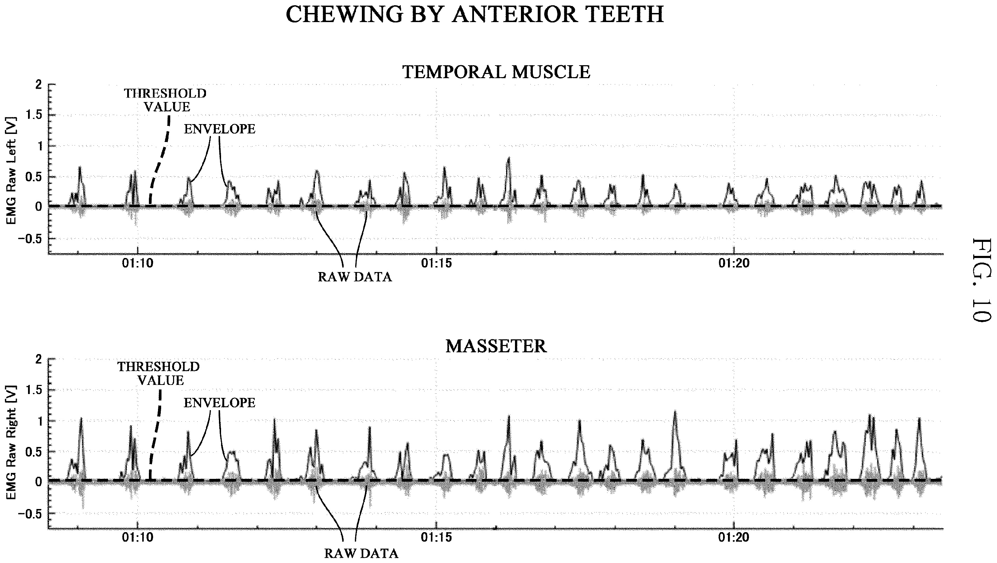

FIG. 9 shows an envelope of muscle activity data of each of temporal muscle and masseter in the case of chewing being performed by using back teeth. As is apparent from FIG. 9, activities of the temporal muscle and the masseter are at the same levels, and both the peak values are similar in each chewing section. FIG. 10 shows envelopes obtained when chewing is performed mainly by anterior teeth, and the activity of the temporal muscle is apparently small and the maximum peak value of the temporal muscle is apparently less than that of the masseter in each chewing section. Thus, by comparing the maximum peak value of the envelope between temporal muscle and masseter in each chewing section, whether or not chewing is performed predominantly on the anterior teeth side in an unbalanced manner can be determined in the analysis.

(Masticatory substance characteristics analysis)

In the present embodiment, the behavior analysis processing unit 22b further incudes a masticatory substance characteristics analysis unit 223 for analyzing characteristics (texture: physical characteristics such as hardness and softness) of a chewed food material. The masticatory substance characteristics analysis unit 223 can analyze the above-described characteristics from a gradient and a duration of a chewing section of an envelope. There is a tendency that the harder the food material is, the greater the gradient in the chewing section is. Characteristics of a masticatory substance, that is, a degree of hardness/softness of the food material can be determined according to the gradient.

The shape in each of chewing sections of envelopes which are obtained from the muscle activity data when a plurality of kinds of foods (prescribed food) having known characteristics are chewed, is stored as a reference shape, and, for example, pattern analysis is performed by using the shape, whereby the characteristics of the masticatory food can be determined. Furthermore, it is also preferable that determination is performed by a machine learning mechanism by using, as training data, the shape (feature point such as a gradient and a peak) of the envelope obtained when a user or the like has chewed the above-described prescribed food.

(Occlusal force analysis unit)

In the present embodiment, the behavior analysis processing unit 22b further includes an occlusal force analysis unit 224 for analyzing an occlusal force at the time of chewing. Relationship between muscle activity data and an occlusal force is different depending on a person. That is, even when chewing is performed at the same occlusal force, a value of the muscle activity is different depending on a person. Therefore, in the present embodiment, a correlation table representing correlation between values (the above-described power values) of the muscle activity and values of an occlusal force is previously generated for the user, and stored in the user information storage unit 31.

The correlation table is generated by obtaining muscle activity data (power values) when a user bites through a plurality of kinds of foods (prescribed foods) having known characteristics as described above. The hardness of the prescribed food, that is, an occlusal force required for biting through the prescribed food is obtained as a fixed value. Therefore, the muscle activity data (power value) obtained when the prescribed food is bit through corresponds to the occlusal force value obtained from the food in one-to-one relationship.

Therefore, the occlusal force analysis unit 224 converts a power value (average value or maximum value) of an envelope in a chewing section to an occlusal force by using the correlation table, and can thus analyze the occlusal force at the time of chewing. The graph in FIG. 6 represents occlusal forces obtained from the envelope in FIG. 5. In the present embodiment, a plurality of kinds of prescribed foods are previously chewed and the correlation table is generated. Instead of such a table, a correlation coefficient (proportionality constant) may be obtained from one kind or a plurality of kinds of prescribed foods according to approximation based on the correlation relationship being proportional.

According to a modification of a technique for generating the correlation table, by connecting a muscle activity measurement unit and an occlusal force measurement unit used in combination, correlation between the occlusal force and the muscle activity data may be directly obtained.

(Chewing action analysis)

In the present embodiment, the behavior analysis processing unit 22b further includes a chewing action analysis unit 225 for analyzing a chewing-and-crushing action, a masticating action, a grinding action, and the like. The chewing action analysis unit 225 determines that chewing is performed by a chewing-and-crushing action when the gradient is small and time is long in the chewing section, whereas the chewing action analysis unit 225 determines that chewing is performed by a masticating action when the gradient is great and time is short. In a case where the chewing action is determined based on the data such as a gradient as described above, it is also preferable that one chewing is divided into a plurality of sections, and displacement or the like in a specific section or each section is used to perform analysis in more detail.

If such a chewing-and-crushing action or a masticating action can be analyzed, transition of these chewing actions can be further analyzed. In a meal, the action typically shifts in the order of putting of a food material in the mouth, a chewing-and-crushing action, a masticating action, a grinding or merging action, and a swallowing action. If such an action can be determined, a series of actions from putting of a food material in the mouth to swallowing can be grasped and eating behavior (behavior characteristics) such as a speed and a habit of eating a meal can be determined.

The quality of the chewing behavior determined by the quality determination unit 23 includes, for example, qualities based on determination as to whether the number of chewing times is large or small, whether or not chewing rhythm is proper, whether or not transition of occlusal actions is proper, whether or not an occlusal force is proper, whether or not chewing balance between the left side and the right side is proper, whether or not diet is unbalanced, or whether or not use of masseter is proper.

For example, information as to whether or not a user has improved chewing quality as compared with the her/his previous quality and information as to whether or not the chewing quality is commensurate with the age are preferably obtained so as to be included in the quality, according to the obtained data, previous information for the user in the determination information storage unit 31c, and age-based statistical information. Preferably, the quality determination unit 23 has a machine learning mechanism 23a, and determines the quality of the chewing behavior, with reference to learning results from the machine learning mechanism 23a.

The information extraction unit 24 functions as extraction means. For example, if the chewing quality is not commensurate with the age, the information extraction unit 24 preferably extracts information such as age-based oral cavity function information, and information about a device for growing/improving purpose and a medical specialist based on a residence of the user.

According thereto, it is also preferable that the improvement is suggested so as to, for example, more slowly perform chewing and/or chew harder food.

What the problem is (force, chewing behavior, or a chewing place) can be presented to a user, and a person who cannot make proper use although the user has teeth and a potential for healthy chewing, is supported so as to improve the chewing quality. Furthermore, chewing behavior is considered to be particularly important for children. For example, grating or chattering in chewing can be pointed out to promote improvement. Furthermore, for elderly people, a way of using masticatory muscle is considered to be particularly important, and a kind of masticatory muscle used, a load thereon, and the like can be determined and presented.

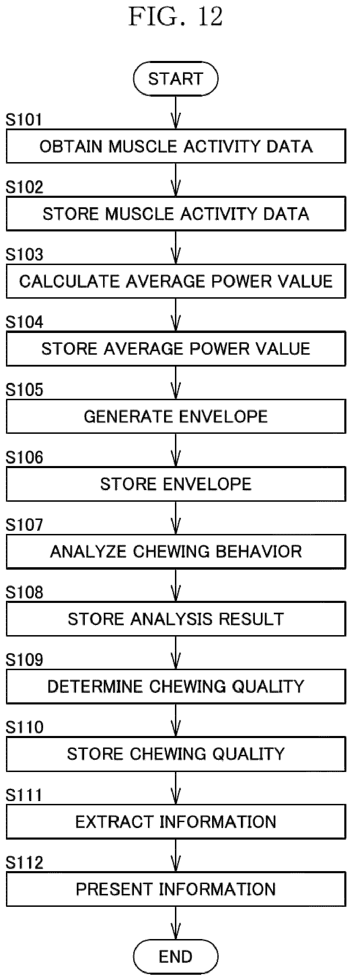

FIG. 12 is a flow chart showing a procedure of processing performed by the chewing assistance system 1 of the present embodiment.

Firstly, the muscle activity obtaining unit 21 obtains, from the muscle activity measurement unit 4, muscle activity data of masticatory muscle of a user at least from putting of the prescribed food (predetermined food) or ordinary food in the mouth up to swallowing (S101), and stores the muscle activity data in the muscle activity data storage unit 31a of the user information storage unit 31 (S102).

Subsequently, the FFT processing unit 22a performs fast Fourier transform of the muscle activity data for each block and thus obtains an average power value in a specific frequency band (S103), stores the average power value in the power value storage unit 311 (S104), generates an envelope of the obtained power value (S105), and stores the envelope in the envelope storage unit 312 (S106).

Subsequently, the behavior analysis processing unit 22b analyzes chewing behavior based on the envelope (S107), and stores the result in the analysis result storage unit 313 (S108). Subsequently, the quality determination unit 23 determines quality of the chewing behavior based on the analysis result (S109), and stores information of the determined quality of the chewing behavior in the determination information storage unit 31c of the user information storage unit 31 (S110).

Subsequently, the information extraction unit 24 receives input of the information of the determined chewing quality and extracts information to be recommended from information of chewing quality stored in the chewing information storage unit 32 (S111). The information output processing unit 25 presents the extracted information to the user by, for example, displaying the information on a display (the information display unit 5) (S112).

Although the embodiment of the present invention has been described above, the present invention is not limited to the embodiment at all. For example, instead of the processing unit being configured by software processing performed by a computer, it is also preferable that a part or the entirety of the processing unit is configured by a hardware processing circuit. In this case, a processing circuit for artificial intelligence can also be used as the machine learning mechanism, and it is needless to say that the present invention can be implemented in various modes, present invention being defined by the appended claims.

INDUSTRIAL APPLICABILITY

The present invention allows quality of complicated chewing behavior having complex aspects to be accurately determined in detail, and allows assistance information based on the determination result to be provided. Therefore, by combining the present invention with instruments and commodities/services for education and training of chewing for children, commodities and services contributing to healthy development of children can be provided. Furthermore, by combining the present invention with cosmetic training instruments and services for, for example, use of masticatory muscle and well-balanced chewing among the anterior, the posterior, the left, and the right sides, cosmetic commodities and services for preventing distortion of the face and obesity, and maintaining vital healthy facial expression can also be provided. Moreover, by combining the present invention with commodities and services for addressing oral frailty such as deterioration of an oral cavity function and weakening of the body for elderly people and the like, commodities and services contributing to extension of healthy life expectancy can also be provided.

DESCRIPTION OF THE REFERENCE CHARACTERS

1 chewing assistance system
2 processing unit
3 storage means
4 muscle activity measurement unit
5 information display unit
10 information processing device
21 muscle activity obtaining unit
22 analysis unit
22a FFT processing unit
22b behavior analysis processing unit
23 quality determination unit
23a machine learning mechanism
24 information extraction unit
25 information output processing unit
31 user information storage unit
31a muscle activity data storage unit
31b chewing behavior storage unit
31c determination information storage unit
32 chewing information storage unit
221 chewing determination unit
222 balance analysis unit
223 masticatory substance characteristics analysis unit
224 occlusal force analysis unit
225 chewing action analysis unit
311 power value storage unit
312 envelope storage unit
313 analysis result storage unit

**Claims**

1. A chewing assistance system (1) comprising an information processing device (10) that includes:

   chewing information storage means (32) that stores information about chewing quality;
   muscle activity obtaining means (21) that is configured to obtain a muscle activity signal of masticatory muscle of a person;
   analysis means (22) that is configured to frequency-analyze the muscle activity signal obtained by the muscle activity obtaining means (21), and to analyze chewing behavior based on the frequency-analyzed muscle activity signal;
   quality determination means (23) that is configured to determine quality of the chewing behavior based on information of the chewing behavior analyzed by the analysis means (22); and
   extraction means (24) that is configured to extract assistance information corresponding to the chewing quality determined by the quality determination means (23), from the chewing information storage means (32),
   wherein the analysis means (22) is configured to frequency-analyze the muscle activity signal and to analyze the chewing behavior based on a change state of a power value in a specific frequency band,
   wherein the analysis means (22) is configured to analyze the chewing behavior based on an envelope obtained by performing, for each block, fast Fourier transform of electromyogram data as the muscle activity signal, by using the envelope as the change state,
   wherein the analysis means (22) is configured to determine that chewing is performed, when the change state indicates a value that exceeds a predetermined threshold value,
   wherein, as to the threshold value, chewing is determined to be performed when an integral value calculated as the change state from the envelope exceeds a predetermined threshold value.

2. The chewing assistance system according to claim 1, wherein the analysis means (22) is configured to analyze chewing balance between a left side and a right side according to the change state of the muscle activity signal of the masticatory muscle on each of the left side and the right side.

**3.** The chewing assistance system according to claim 1 or 2, wherein the analysis means (22) is configured to analyze characteristics of a masticatory substance based on a gradient and a duration of a chewing section in which chewing is determined to be performed from the change state of the envelope.

**4.** The chewing assistance system according to any one of claims 1 to 3, comprising

a user information storage unit (31) that is configured to store correlation between values of an occlusal force and values of a muscle activity of a user, the correlation being acquired by obtaining a value of a muscle activity during eating of prescribed food having such known characteristics that an occlusal force required for biting-through is obtained as a fixed value, wherein the analysis means (22) is configured to analyze the occlusal force during chewing, based on the correlation and a value in a chewing section in which chewing is determined to be performed from the change state of the envelope.

**5.** The chewing assistance system according to any one of claims 1 to 4, wherein

the analysis means (22) includes a machine learning mechanism, and
the chewing behavior is determined with reference to a learning result from the machine learning mechanism.

**6.** The chewing assistance system according to any one of claims 1 to 5, wherein

the chewing behavior analyzed by the analysis means (22)
includes behavior representing at least one of a total number of chewing times, chewing rhythm, transition of occlusal actions during a meal, an occlusal force level, chewing balance between anterior and posterior sides/between left and right sides, and characteristics of a masticatory substance.

**7.** The chewing assistance system according to any one of claims 1 to 6, wherein the quality of the chewing behavior to be determined by the quality determination means (23) includes quality based on at least one of determinations as to whether a total number of chewing times is large or small, whether chewing rhythm is proper, whether transition of occlusal actions is proper, whether an occlusal force is proper, whether chewing balance between a left side and a right side is proper, whether diet is unbalanced, and whether use of masseter is proper.

**8.** The chewing assistance system according to any one of claims 1 to 7, wherein

the quality determination means (23)
compares a chewing behavior with a previous chewing behavior of a same person and determines whether the chewing behavior has improved.

**9.** The chewing assistance system according to any one of claims 1 to 8, wherein

the quality determination means (23) has a machine learning mechanism, and
the quality of the chewing behavior is determined with reference to a learning result from the machine learning mechanism.

**10.** A chewing assistance program comprising a control program for causing an information processing device (10) to function as the chewing assistance system (1) according to any one of claims 1 to 9, the chewing assistance program causing the information processing device (10) to function as the muscle activity obtaining means (21), the analysis means (22), the quality determination means (23), and the extraction means (24).

**Patentansprüche**

**1.** Kauhilfesystem (1) mit einer Informationsverarbeitungsvorrichtung (10), die aufweist:

eine Kauinformations-Speichereinrichtung (32), die Informationen über die Kauqualität speichert;
eine Muskelaktivitäts-Bezugseinrichtung (21), die so konfiguriert ist, dass sie ein Muskelaktivitätssignal des Kaumuskels einer Person bezieht;
eine Analyseneinrichtung (22), die so konfiguriert ist, dass sie das durch die Muskelaktivitäts-Bezugseinrichtung (21) bezogene Muskelaktivitätssignal frequenzanalysiert und das Kauverhalten auf der Grundlage des frequenzanalysierten Muskelaktivitätssignals analysiert;
eine Qualitätsbestimmungseinrichtung (23), die so konfiguriert ist, dass sie die Qualität des Kauverhaltens auf der Grundlage von Informationen über das durch die Analyseneinrichtung (22) analysierte Kauverhalten bestimmt; und
eine Extraktionseinrichtung (24), die so konfiguriert ist, dass sie Hilfeinformationen, die der durch die Qualitätsbestimmungseinrichtung (23) bestimmten Kauqualität entsprechen, aus der Kauinformations-Speichereinrichtung (32) extrahiert,

wobei die Analyseneinrichtung (22) so konfiguriert ist, dass sie das Muskelaktivitätssignal frequenzanalysiert und das Kauverhalten auf der Grundlage eines Änderungszustands eines Leistungswerts in einem spezifischen Frequenzband analysiert, wobei die Analyseneinrichtung (22) so konfiguriert ist, dass sie das Kauverhalten auf der Grundlage einer Hüllkurve analysiert, die durch für jeden Block erfolgendes Durchführen einer schnellen Fouriertransformation von Elektromyogrammdaten als Muskelaktivitätssignal bezogen werden, indem sie die Hüllkurve als den Änderungszustand verwendet,

wobei die Analyseneinrichtung (22) so konfiguriert ist, dass sie bestimmt, dass Kauen durchgeführt wird, wenn der Änderungszustand einen Wert anzeigt, der einen vorbestimmten Schwellwert überschreitet,

wobei bezogen auf den Schwellwert bestimmt wird, dass Kauen durchgeführt wird, wenn ein als der Änderungszustand anhand der Hüllkurve berechneter Integralwert einen vorbestimmten Schwellwert überschreitet.

2. Kauhilfesystem nach Anspruch 1, wobei die Analyseneinrichtung (22) so konfiguriert ist, dass sie das Kaugleichgewicht zwischen einer linken Seite und einer rechten Seite gemäß dem Änderungszustand des Muskelaktivitätssignals des Kaumuskels jeweils auf der linken Seite und der rechten Seite analysiert.

3. Kauhilfesystem nach Anspruch 1 oder 2, wobei die Analyseneinrichtung (22) so konfiguriert ist, dass sie Kennwerte einer Kaumasse auf der Grundlage eines Gradienten und einer Dauer eines Kauabschnitts, in dem bestimmt wird, dass das Kauen durchgeführt wird, anhand des Änderungszustands der Hüllkurve analysiert.

4. Kauhilfesystem nach einem der Ansprüche 1 bis 3, das aufweist:

   eine Benutzerinformations-Speichereinheit (31), die so konfiguriert ist, dass sie die Korrelation zwischen Werten einer Okklusionskraft und Werten einer Muskelaktivität eines Benutzers speichert, wobei die Korrelation durch Beziehen eines Werts einer Muskelaktivität während des Essens vorgeschriebener Nahrungsmittel mit solchen bekannten Kennwerten erfasst wird, dass eine zum Durchbeißen erforderliche Okklusionskraft als Festwert bezogen wird, wobei

   die Analyseneinrichtung (22) so konfiguriert ist, dass sie die Okklusionskraft während des Kauens auf der Grundlage der Korrelation und eines Werts in einem Kauabschnitt bezieht, in dem anhand des Änderungszustands der Hüllkurve bestimmt wird, dass das Kauen durchgeführt wird.

5. Kauhilfesystem nach einem der Ansprüche 1 bis 4, wobei

   die Analyseneinrichtung (22) einen Maschinenlernmechanismus aufweist und
   das Kauverhalten mit Bezug auf ein Lernergebnis vom Maschinenlernmechanismus bestimmt wird.

6. Kauhilfesystem nach einem der Ansprüche 1 bis 5, wobei
   das durch die Analyseneinrichtung (22) analysierte Kauverhalten mindestens eines der folgenden Verhalten aufweist: eine Gesamtzahl von Kauvorgängen; Kaurhythmus; Übergang von Okklusionsaktionen während einer Mahlzeit; einen Okklusionskraftpegel; ein Kaugleichgewicht zwischen anterior und posterior gelegener Seite/zwischen linker und rechter Seite; und Kennwerte einer Kaumasse.

7. Kauhilfesystem nach einem der Ansprüche 1 bis 6, wobei die durch die Qualitätsbestimmungseinrichtung (23) zu bestimmende Qualität des Kauverhaltens mindestens eine der folgenden Qualitäten aufweist: ob eine Gesamtzahl von Kauvorgängen groß oder klein ist; ob der Kaurhythmus richtig ist; ob der Übergang von Okklusionsaktionen richtig ist; ob eine Okklusionskraft richtig ist; ob das Kaugleichgewicht zwischen einer linken Seite und einer rechten Seite richtig ist; ob die Diät unausgeglichen ist; und ob der Gebrauch des Masseters richtig ist.

8. Kauhilfesystem nach einem der Ansprüche 1 bis 7, wobei
   die Qualitätsbestimmungseinrichtung (23) ein Kauverhalten mit einem früheren Kauverhalten derselben Person vergleicht und bestimmt, ob sich das Kauverhalten verbessert hat.

9. Kauhilfesystem nach einem der Ansprüche 1 bis 8, wobei

   die Qualitätsbestimmungseinrichtung (23) einen Maschinenlernmechanismus hat und
   die Qualität des Kauverhaltens in Bezug auf ein Lernergebnis vom Maschinenlernmechanismus bestimmt wird.

10. Kauhilfeprogramm mit einem Steuerprogramm zum Veranlassen, dass eine Informationsverarbeitungsvorrichtung (10) als das Kauhilfesystem (1) nach einem der Ansprüche 1 bis 9 fungiert, wobei das Kauhilfeprogramm die Informationsverarbeitungsvorrichtung (10) veranlasst, als die Muskelaktivitäts-Bezugseinrichtung (21), die Analyseneinrichtung (22), die Qualitätsbestimmungseinrichtung (23) und die Extraktionseinrichtung (24) zu fungieren.

## Revendications

1. Système d'assistance à la mastication (1) comprenant un dispositif de traitement d'informations (10) qui comporte :

   un moyen de stockage d'informations de mastication (32) qui stocke des informations concernant la qualité de la mastication ;
   un moyen d'obtention d'activité musculaire (21) qui est configuré pour obtenir un signal d'activité musculaire d'un muscle masticatoire d'une personne ;
   un moyen d'analyse (22) qui est configuré pour analyser en fréquence le signal d'activité musculaire obtenu par le moyen d'obtention d'activité musculaire (21) et pour analyser le comportement de mastication sur la base du signal d'activité musculaire analysé en fréquence ;
   un moyen de détermination de qualité (23) qui est configuré pour déterminer la qualité du comportement de mastication sur la base d'informations du comportement de mastication analysé par le moyen d'analyse (22) ; et
   un moyen d'extraction (24) qui est configuré pour extraire des informations d'assistance correspondant à la qualité de mastication déterminée par le moyen de détermination de qualité (23), à partir du moyen de stockage d'informations de mastication (32),
   dans lequel le moyen d'analyse (22) est configuré pour analyser en fréquence le signal d'activité musculaire et pour analyser le comportement de mastication sur la base d'un état de changement d'une valeur de puissance dans une bande de fréquence spécifique,
   dans lequel le moyen d'analyse (22) est configuré pour analyser le comportement de mastication sur la base d'une enveloppe obtenue par mise en œuvre, pour chaque bloc, d'une transformée de Fourier rapide de données d'électromyogramme en tant que signal d'activité musculaire, par utilisation de l'enveloppe en tant qu'état de changement,
   dans lequel le moyen d'analyse (22) est configuré pour déterminer qu'une mastication est effectuée, quand l'état de changement indique une valeur qui dépasse une valeur de seuil prédéterminée,
   dans lequel, en ce qui concerne la valeur de seuil, il est déterminé qu'une mastication est effectuée quand la valeur intégrale calculée en tant qu'état de changement à partir de l'enveloppe dépasse une valeur de seuil prédéterminée.

2. Système d'assistance à la mastication selon la revendication 1, dans lequel le moyen d'analyse (22) est configuré pour analyser l'équilibre de mastication entre le côté gauche et le côté droit en fonction de l'état de changement du signal d'activité musculaire du muscle masticatoire sur chacun parmi le côté gauche et le côté droit.

3. Système d'assistance à la mastication selon la revendication 1 ou 2, dans lequel le moyen d'analyse (22) est configuré pour analyser des caractéristiques d'une substance masticatoire sur la base du gradient et de la durée d'une section de mastication dans laquelle il est déterminé qu'une mastication est effectuée à partir de l'état de changement de l'enveloppe.

4. Système d'assistance à la mastication selon l'une quelconque des revendications 1 à 3, comprenant une unité de stockage d'informations utilisateur (31) qui est configurée pour stocker une corrélation entre les valeurs de force occlusale et les valeurs d'activité musculaire d'un utilisateur, la corrélation étant acquise par obtention d'une valeur d'activité musculaire durant la consommation d'un aliment prescrit ayant des caractéristiques connues telles que la force occlusale requise pour mordre à travers soit obtenue sous la forme d'une valeur fixe, dans lequel le moyen d'analyse (22) est configuré pour analyser la force occlusale pendant la mastication, sur la base de la corrélation et d'une valeur dans une section de mastication dans laquelle il est déterminé qu'une mastication est effectuée à partir de l'état de changement de l'enveloppe.

5. Système d'assistance à la mastication selon l'une quelconque des revendications 1 à 4, dans lequel

   le moyen d'analyse (22) comprend un mécanisme d'apprentissage machine, et
   le comportement de mastication est déterminé en référence à un résultat d'apprentissage à partir du mécanisme d'apprentissage machine.

6. Système d'assistance à la mastication selon l'une quelconque des revendications 1 à 5, dans lequel le comportement de mastication analysé par le moyen d'analyse (22) comprend un comportement représentant au moins l'un parmi le nombre de total de mastications, le rythme de mastication, la transition d'actions occlusales pendant un repas, le niveau de force occlusale, l'équilibre de mastication entre les côtés antérieur et postérieur / entre les côtés gauche et droit, et les caractéristiques d'une substance masticatoire.

7. Système d'assistance à la mastication selon l'une quelconque des revendications 1 à 6, dans lequel la qualité du comportement de mastication devant être déterminée par le moyen de détermination de qualité (23) comprend la qualité basée sur au moins l'une des déterminations que le nombre total de mastications est grand ou petit, que le rythme de mastication est ou non approprié, que la transition d'actions occlusales est ou non appropriée, que la force occlusale est ou non appropriée, que l'équilibre de mastication entre le côté gauche et le côté droit est ou non approprié, que le régime alimentaire est ou non déséquilibré, et que l'utilisation du masséter est ou non appropriée.

8. Système d'assistance à la mastication selon l'une quelconque des revendications 1 à 7, dans lequel le moyen de détermination de qualité (23) compare le comportement de mastication avec le comportement de mastication antérieur d'une même personne et détermine si le comportement de mastication s'est ou non amélioré.

9. Système d'assistance à la mastication selon l'une quelconque des revendications 1 à 8, dans lequel

le moyen de détermination de qualité (23) a un mécanisme d'apprentissage machine, et
la qualité du comportement de mastication est déterminée en référence à un résultat d'apprentissage provenant du mécanisme d'apprentissage machine.

10. Programme d'assistance à la mastication comprenant un programme de commande pour amener un dispositif de traitement d'informations (10) à fonctionner comme le système d'assistance à la mastication (1) de l'une quelconque des revendications 1 à 9, le programme d'assistance à la mastication amenant le dispositif de traitement d'informations (10) à fonctionner en tant que moyen d'obtention d'activité musculaire (21), moyen d'analyse (22), moyen de détermination de qualité (23), et moyen d'extraction (24).

FIG. 1

PROCESSING UNIT — 2

MUSCLE ACTIVITY OBTAINING UNIT — 21

ANALYSIS UNIT — 22

FFT PROCESSING UNIT — 22a

BEHAVIOR ANALYSIS PROCESSING UNIT — 22b

CHEWING DETERMINATION UNIT — 221

BALANCE ANALYSIS UNIT — 222

MASTICATORY SUBSTANCE CHARACTERISTICS ANALYSIS UNIT — 223

OCCLUSAL FORCE ANALYSIS UNIT — 224

CHEWING ACTION ANALYSIS UNIT — 225

QUALITY DETERMINATION UNIT — 23

MACHINE LEARNING MECHANISM — 23a

INFORMATION EXTRACTION UNIT — 24

INFORMATION OUTPUT PROCESSING UNIT — 25

STORAGE MEANS — 3

USER INFORMATION STORAGE UNIT — 31

MUSCLE ACTIVITY DATA STORAGE UNIT — 31a

CHEWING BEHAVIOR STORAGE UNIT — 31b

POWER VALUE STORAGE UNIT — 311

ENVELOPE STORAGE UNIT — 312

ANALYSIS RESULT STORAGE UNIT — 313

DETERMINATION INFORMATION STORAGE UNIT — 31c

CHEWING INFORMATION STORAGE UNIT — 32

MUSCLE ACTIVITY MEASUREMENT UNIT — 4

INFORMATION DISPLAY UNIT — 5

10 (1)

EP 4 082 438 B1

FIG. 2A

ONE CHEWING

rawEMG L X 0.03125

FIG. 2B

raw EMG R X 0.03125

ONE CHEWING

FIG. 3A

FIG. 3B

# FIG. 4

ENVELOPE

THRESHOLD VALUE

BACKGROUND

0

64ms — | 64ms — | 5s | (t)

START OF CHEWING

STOP OF CALCULATION
OF THRESHOLD VALUE

END OF
CHEWING

RESTART OF CALCULATION
OF THRESHOLD VALUE

# FIG. 5

THRESHOLD VALUE    ENVELOPE

0.75

0.5

0.25

0

−0.25

EMG Raw Left [V]

A

00:30            00:35            00:40

ONE CHEWING                RAW DATA

## FIG. 6

OCCLUSAL FORCE (N)

A'

INTEGRATION
THRESHOLD VALUE

VALUE (Vsec) OF SURFACE INTEGRAL
FOR ENVELOPE

VALUE (Vsec) OF SURFACE INTEGRAL
FOR RAW DATA

## FIG. 7

### CHEWING ON LEFT SIDE

(LEFT)

THRESHOLD VALUE · ENVELOPE · RAW DATA

(RIGHT)

THRESHOLD VALUE · ENVELOPE · RAW DATA

# FIG. 8

**CHEWING ON RIGHT SIDE**

# CHEWING BY BACK TEETH

## TEMPORAL MUSCLE

THRESHOLD VALUE

ENVELOPE

RAW DATA

00:30

00:35

EMG Raw Left [V]

2
1.5
1
0.5
0
−0.5

## MASSETER

THRESHOLD VALUE

ENVELOPE

RAW DATA

00:30

00:35

EMG Raw Right [V]

2
1.5
1
0.5
0
−0.5

FIG. 9

FIG. 10

CHEWING BY ANTERIOR TEETH

# FIG. 11A

FIG. 11B

# FIG. 12

```
                    ( START )
                        │
    S101                ▼
    ┌─────────────────────────────────────────┐
    │      OBTAIN MUSCLE ACTIVITY DATA         │
    └─────────────────────────────────────────┘
                        │
    S102                ▼
    ┌─────────────────────────────────────────┐
    │       STORE MUSCLE ACTIVITY DATA         │
    └─────────────────────────────────────────┘
                        │
    S103                ▼
    ┌─────────────────────────────────────────┐
    │      CALCULATE AVERAGE POWER VALUE       │
    └─────────────────────────────────────────┘
                        │
    S104                ▼
    ┌─────────────────────────────────────────┐
    │       STORE AVERAGE POWER VALUE          │
    └─────────────────────────────────────────┘
                        │
    S105                ▼
    ┌─────────────────────────────────────────┐
    │           GENERATE ENVELOPE              │
    └─────────────────────────────────────────┘
                        │
    S106                ▼
    ┌─────────────────────────────────────────┐
    │            STORE ENVELOPE                │
    └─────────────────────────────────────────┘
                        │
    S107                ▼
    ┌─────────────────────────────────────────┐
    │        ANALYZE CHEWING BEHAVIOR          │
    └─────────────────────────────────────────┘
                        │
    S108                ▼
    ┌─────────────────────────────────────────┐
    │          STORE ANALYSIS RESULT           │
    └─────────────────────────────────────────┘
                        │
    S109                ▼
    ┌─────────────────────────────────────────┐
    │        DETERMINE CHEWING QUALITY         │
    └─────────────────────────────────────────┘
                        │
    S110                ▼
    ┌─────────────────────────────────────────┐
    │          STORE CHEWING QUALITY           │
    └─────────────────────────────────────────┘
                        │
    S111                ▼
    ┌─────────────────────────────────────────┐
    │           EXTRACT INFORMATION            │
    └─────────────────────────────────────────┘
                        │
    S112                ▼
    ┌─────────────────────────────────────────┐
    │          PRESENT INFORMATION             │
    └─────────────────────────────────────────┘
                        │
                        ▼
                    (  END  )
```

**EP 4 082 438 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H698865 A
- JP 2019047859 A
- WO 2018146672 A1
- WO 2019230528 A1
- US 2017265978 A1

**Non-patent literature cited in the description**

- **KOBAYASHI YOSHINORI** ; **IRAI RONBUN**. Kogo/-soshaku ga tsukuru kenkojumyo. *Nichihotetsukaishi Ann Jpn Prosthodont*, 2011, 189-219
- **YOSHINORI KOBAYASHI**. A long Life Built by Mastication and Occlusion. *Annals of Japan Prosthodontic Society*, 2011, 189-219